# EUROPÄISCHE PATENTSCHRIFT

(11) **EP 1 000 631 B1**
(45) Veröffentlichungstag und Bekanntmachung des Hinweises auf die Patenterteilung: **10.07.2002**
(21) Anmeldenummer: 00100878.8
(22) Anmeldetag: 04.10.1997
(51) Int. Cl.: A61M 5/24, A61M 5/315

(54) **Injektionsgerät**
Injection device
Appareil d'injection

(30) Priorität: 03.03.1997 DE 29703820 U
(43) Veröffentlichungstag der Anmeldung: 17.05.2000
(62) Teilanmeldung aus: 97912108.4
(73) Patentinhaber: B D Medico S.a.r.l., 1295 Mies (CH)
(72) Erfinder: Gabriel, Jochen, 70192 Stuttgart (DE)
(74) Vertreter: Raible, Hans, Dipl.-Ing.

(56) Entgegenhaltungen:
- EP-A- 0 327 910
- DE-A- 19 519 147
- US-A- 3 905 366
- US-A- 5 295 976

## Beschreibung

Die Erfindung betrifft ein Injektionsgerät zum Injizieren einer einstellbaren Dosis einer Injektionsflüssigkeit.

Aus der EP-A1-0 295 075 kennt man ein manuelles Injektionsgerät, das zum Auspressen der gewünschten Dosis eine Zahnstange verwendet. Vor dem Injizieren wird zunächst die Dosisanzeige auf Null zurückgedreht. Dann werden durch Drehen eines Gehäuseteils Klemmbacken gelöst, also außer Eingriff mit der Zahnstange gebracht. Dabei geraten die Klemmbacken in Eingriff mit einer Hülse. Letztere wird nun durch Drehen eines Gehäuseteils axial verschoben, um die gewünschte Dosis einzustellen. Dann werden die Klemmbacken wieder mit der Zahnstange in Eingriff gebracht. Jetzt wird die Nadel eingestochen, und durch Druck auf einen Betätigungsknopf wird die eingestellte Dosis injiziert. Das Einstellen der Dosis ist bei diesem Gerät sehr kompliziert.

Ein ähnliches Gerät ist bekannt aus der EP-A1-0 498 737. Bei diesem ist die Bedienung dadurch vereinfacht, dass beim Injizieren eine Feder gespannt wird, die anschließend einen Teil der bei der EP-A1-0 295 075 beschriebenen Vorgänge automatisch ablaufen lässt, sofern keine Änderung der eingestellten Dosis vorgenommen wird. Eine Dosisänderung erfordert jedoch auch hier komplizierte Schritte.

Es ist deshalb eine Aufgabe der Erfindung, ein neues Injektionsgerät bereitzustellen.

Nach der Erfindung wird diese Aufgabe gelöst durch den Gegenstand des Patentanspruchs 1. Es ergibt sich hier eine einfache Dosiseinstellung, da der Benutzer zur Dosiseinstellung nur proximales Gehäuseteil und distales Gehäuseteil relativ zueinander verdrehen muss.

Eine bevorzugte Weiterbildung ist Gegenstand des Anspruchs 2, wobei ein solches Gerät in besonders bevorzugter Weise gemäß Anspruch 3 ausgebildet wird, da man hierdurch in einfacher Weise durch die beiden Fenster hindurch die eingestellte Dosis ablesen kann.

Weitere Einzelheiten und vorteilhafte Weiterbildungen der Erfindung ergeben sich aus den im folgenden beschriebenen und in der Zeichnung dargestellten, in keiner Weise als Einschränkung der Erfindung zu verstehenden Ausführungsbeispielen, sowie aus den Unteransprüchen. Es zeigt:
- Fig. 1: eine Darstellung einer Ausführungsform eines erfindungsgemäßen Injektionsgeräts, etwa in natürlicher Größe,
- Fig. 2: eine vergrößerte Darstellung der Einzelheit A der Fig. 1,
- Fig. 3: eine Darstellung analog Fig. 1, aber in vergrößerter Darstellung und im Längsschnitt,
- Fig. 4: eine vergrößerte Darstellung der Einzelheit B der Fig. 3,
- Fig. 5: eine Darstellung analog Fig. 1 und 3, wobei der proximale Teil im Längsschnitt dargestellt ist,
- Fig. 6: eine vergrößerte Darstellung der Einzelheit C der Fig. 5,
- Fig. 7: eine Darstellung entsprechend Fig. 4; diese zeigt das Injektionsgerät nach Abschluß einer injektion,
- Fig. 8: den Beginn der Vorbereitung für eine nachfolgende Injektion,
- Fig. 9: den weiteren Verlauf einer solchen Vorbereitung
- Fig. 10: den Abschluß einer Injektionsvorbereitung; das Gerät ist jetzt für eine Injektion mit der vorgewählten Dosis bereit,
- Fig. 11: den Beginn einer Injektion, die manuell erfolgt (Kraft K'),
- Fig. 12: den Abschluß einer Injektion,
- Fig. 13: einen Längsschnitt durch ein erfindungsgemäßes Injektionsgerät in einer zur Injektion bereiten Stellung,
- Fig. 14: eine vergrößerte Darstellung der Einzelheit D der Fig. 13,
- Fig. 15: einen Längsschnitt durch einen Teil des Gehäuses des Injektionsgeräts einschließlich eines im Gehäuse drehbar gelagerten Teils zur Aufnahme eines Behälters mit Injektionsflüssigkeit,
- Fig. 16: einen Längsschnitt durch das Auspreßglied und das Betätigungsglied,
- Fig. 17: einen Schnitt, gesehen längs der Linie XVII-XVII der Fig. 15,
- Fig. 18: einen Schnitt, gesehen längs der Linie XVIII-XVIII der Fig. 16,
- Fig. 19: eine Darstellung zur Erläuterung einer bevorzugten Weiterbildung der Erfindung,
- Fig. 20: eine Darstellung von Elementen für die Dosiseinstellung und - anzeige, und zwar bei der vorgewählten Dosis Null, und
- Fig. 21: eine Darstellung analog Fig. 20, aber für die maximal einstellbare Dosis.

In der nachfolgenden Beschreibung werden die Begriffe proximal und distal in der in der Medizin gebräuchlichen Weise benutzt, also:
- Proximal:: Dem Patienten zugewandt (dies ist die Seite des Injektionsgeräts mit der Nadel).
- Distal:: Vom Patienten abgewandt.

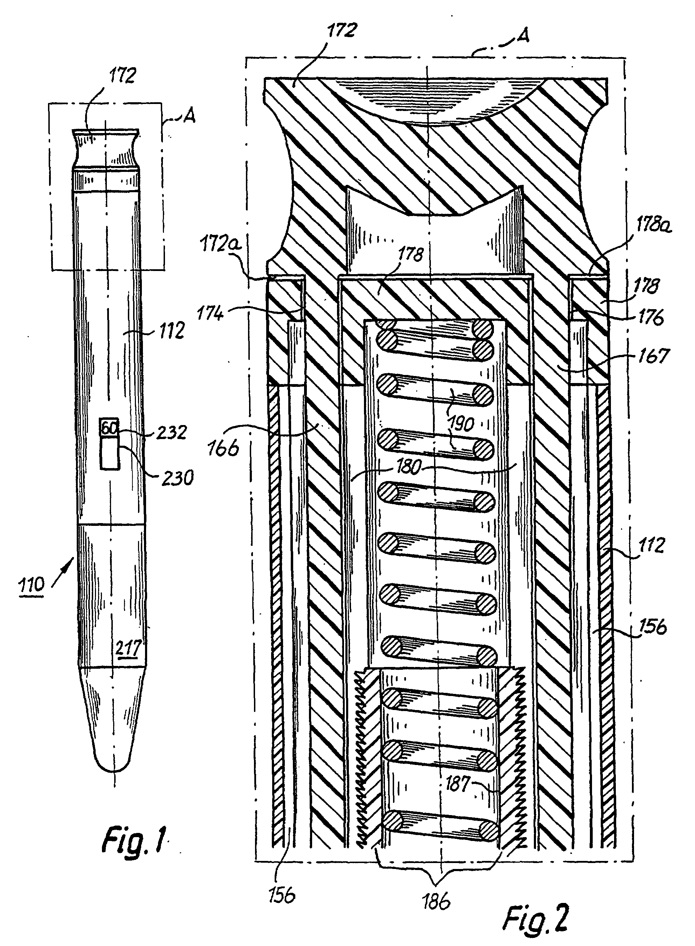

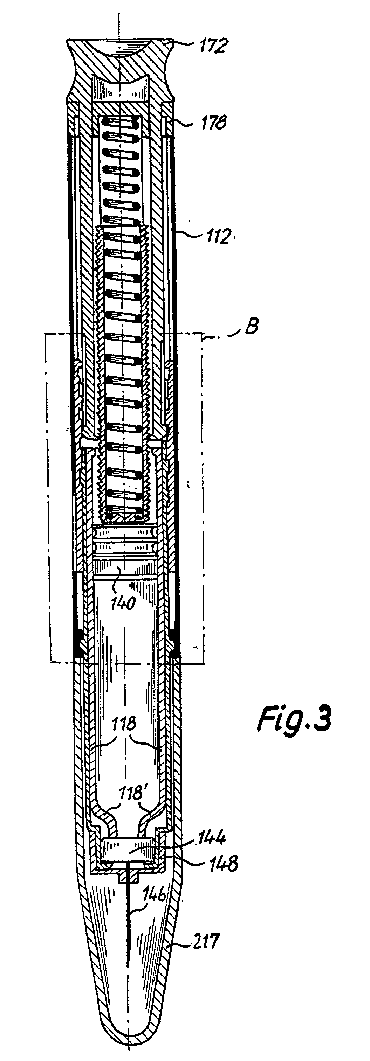

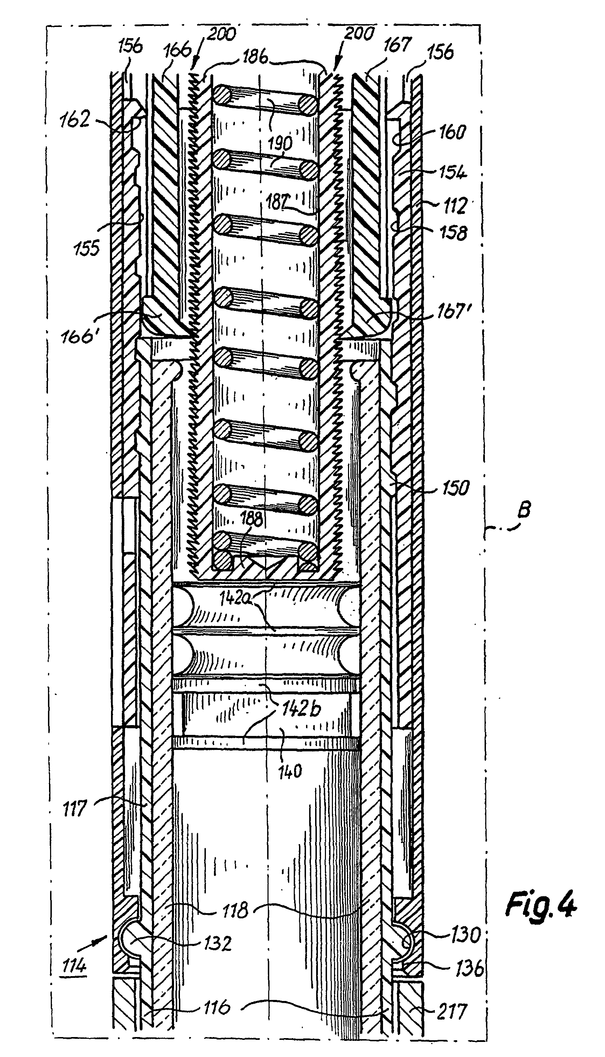

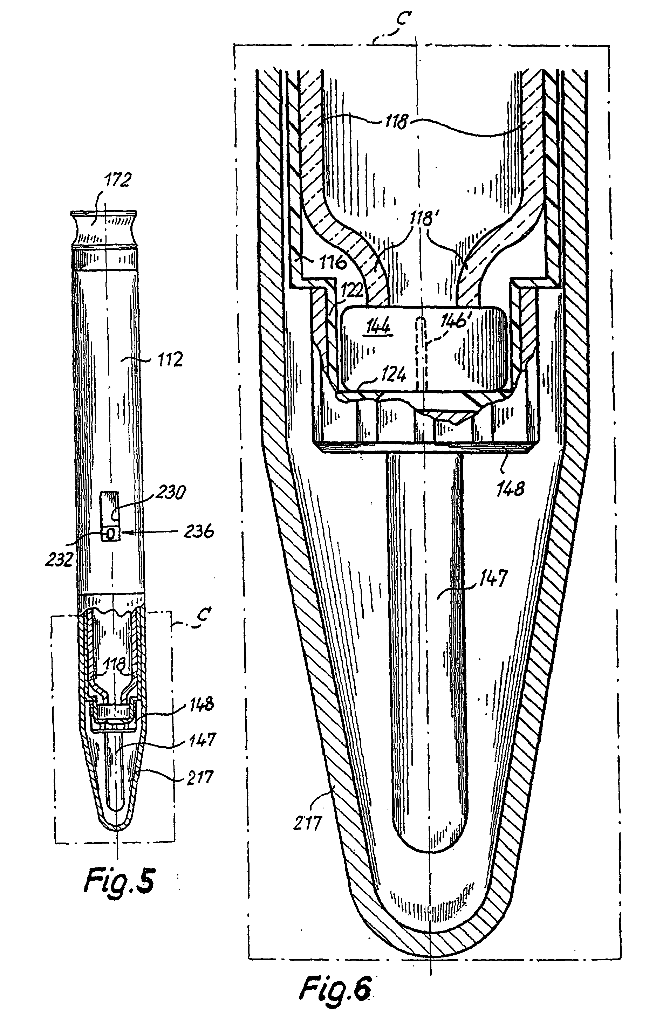

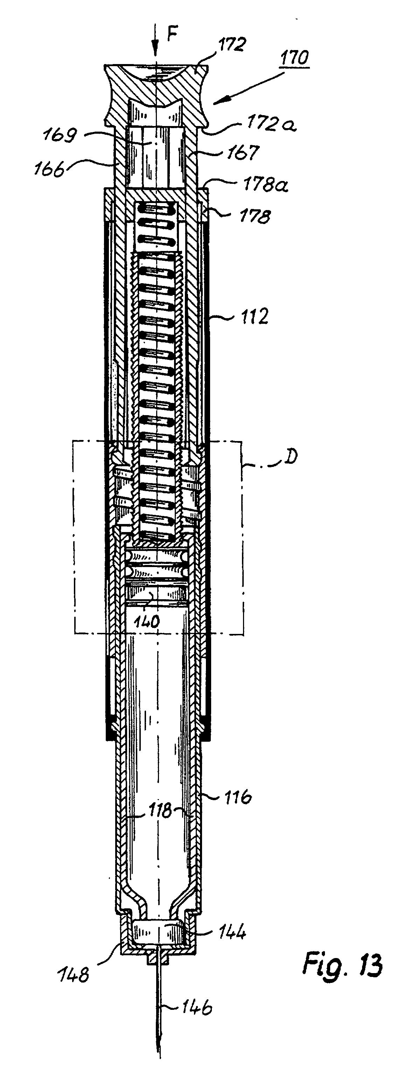

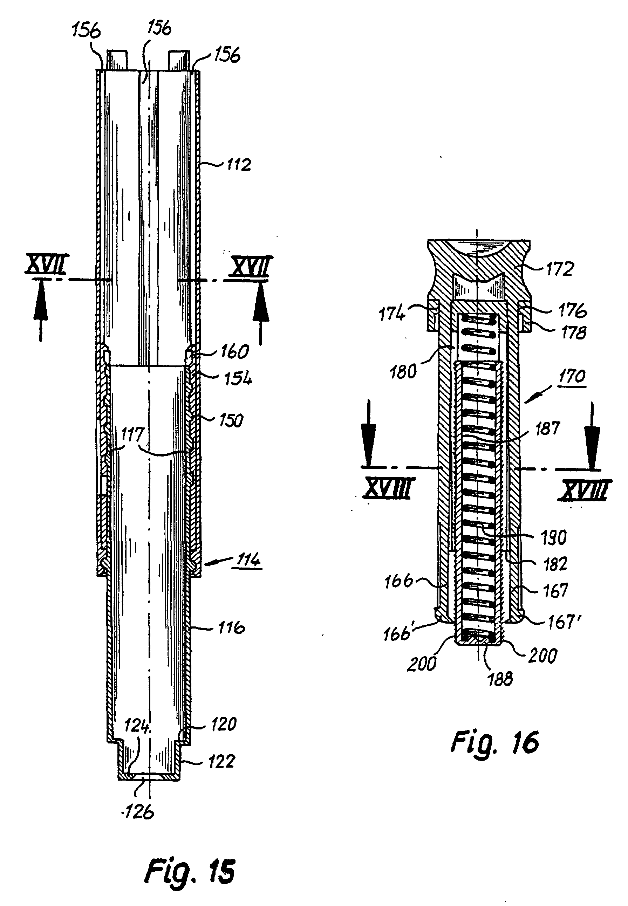

Wie Fig. 15 zeigt, weist das Gehäuse des in Fig. 1 dargestellten Injektionsgeräts 110 einen distalen Abschnitt 112 nach Art eines Rohres aus einem geeigneten Kunststoff auf, und mit diesem Abschnitt oder Gehäuseteil 112 ist an dessen proximalem Ende über ein Lager 114 ein ebenfalls rohrartiges Teil 116 drehbar verbunden, das zur Aufnahme eines Behälters 118 (Fig. 3, 4) mit der zu injizierenden Flüssigkeit dient und deshalb an seinem proximalen Ende mit einer Schulter 120 und einem kurzen zylindrischen Abschnitt 122 kleineren Durchmessers versehen ist, der an seinem proximalen Ende ebenfalls eine Schulter 124 aufweist, die von einer zentralen Öffnung 126 durchdrungen ist. Der zylindrische Abschnitt 122 kann auf seiner Außenseite mit einem Gewinde zur Befestigung eines Kanülenträgers 148 versehen sein, wie er z.B. in Fig. 6 oder 13 zusammen mit einer Kanüle (Injektionsnadel) 146 dargestellt ist. Im unbenutzten Zustand befindet sich gemäß Fig. 1 über dem proximalen Abschnitt des rohrartigen Teils 116 eine Schutzkappe 217, welche diesen Abschnitt steril abdeckt und vor Verunreinigung schützt.

Das Lager 114 hat, wie in Fig. 4 dargestellt, eine Ringnut 130 am proximalen Ende des Gehäuseteils 112, in die ein hierzu komplementärer Wulst 132 des Gehäuseteils 116 eingeclipst ist, so daß dieses Lager 114 als Axial- und Radiallager wirkt. Zum Einclipsen schließt sich an die Ringnut 130 auf deren proximaler Seite ein Abschnitt 136 vergrößerten Innendurchmessers an, der sich kegelförmig in proximaler Richtung erweitert.

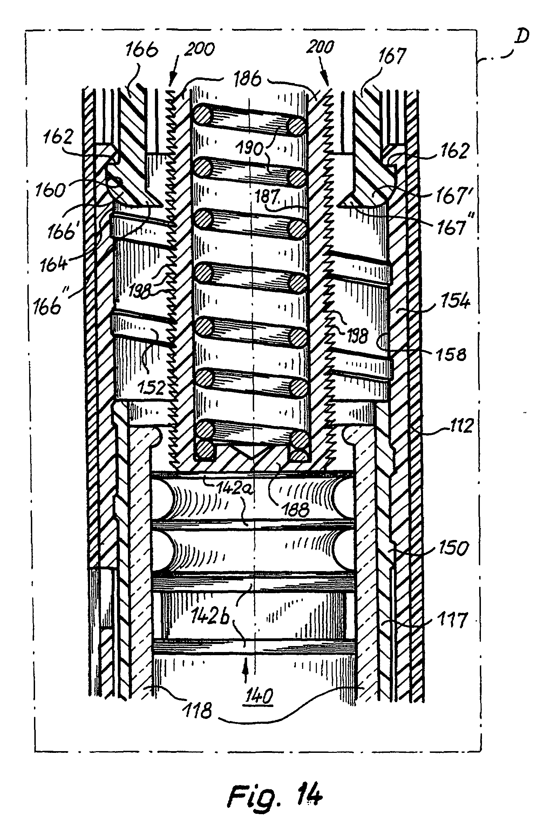

Der Behälter 118 ist eine sogenannte Karpule, die z.B. 1,5 ml oder 3 ml Injektionsflüssigkeit enthalten kann, z.B. Wachstumshormon oder Insulin. Er besteht gewöhnlich aus Glas und hat an seinem distalen Ende einen Kolben 140, der z.B. die in Fig. 14 dargestellte Form mit mehreren Umfangsrippen 142a, 142b haben kann, die mit Vorspannung gegen die Innenseite des Behälters 118 anliegen, also mit entsprechender Reibung. Um einen solchen Kolben 140 relativ zum Behälter 118 zu verschieben, ist also eine bestimmte Minimalkraft erforderlich. Erst wenn die Kraft auf den Kolben 140 diesen Wert übersteigt, bewegt er sich relativ zum Behälter 118.

Der Behälter 118 hat auf seiner proximalen Seite einen verjüngten Hals 118', vgl. Fig. 3, auf dem in der üblichen Weise mittels einer Metallkappe 144 eine (nicht dargestellte) dünne Gummimembran befestigt ist. Die in Fig. 13 dargestellte Injektionsnadel (Kanüle) 146 ist an einem Kanülenträger 148 befestigt, der auf den zylindrischen Abschnitt 122 (Fig. 6 und 15) aufgesteckt oder aufgeschraubt werden kann. Die Nadel 146 hat ein distales Ende 146' (Fig. 6), das die beschriebene Gummimembran (in der Kappe 144) durchsticht, so daß Flüssigkeit aus dem Behälter 118 durch die Nadel 146 nach außen gepreßt werden kann, wenn der Kolben 140 in Fig. 13 in proximaler Richtung, also nach unten, bewegt wird. Derartige Karpulen 118 und Nadeln 146 werden in großen Stückzahlen hergestellt und sind dem Fachmann dieses Gebiets vertraut.

Wie z.B. Fig. 13 zeigt, kann das proximale Gehäuseteil 116 mitsamt dem in ihm enthaltenen Behälter 118 relativ zum distalen Gehäuseteil 112 verdreht werden. Diese Verdrehung dient der Vorwahl einer Injektionsdosis, z.B. von 4 Insulineinheiten, und diese Dosis, einmal eingestellt, bleibt bei den folgenden Injektionen unverändert, sofern sie nicht vom Patienten, seinem Arzt, oder seiner Krankenschwester neu eingestellt wird. Diese Dosis wird also gewöhnlich nur einmal eingestellt, und wenn z.B. einmal vier Einheiten eingestellt wurden, wird bei allen nachfolgenden Injektionen - ohne neue Einstellung - eine Dosis von vier Einheiten injiziert, bis die Karpule 118 leer ist.

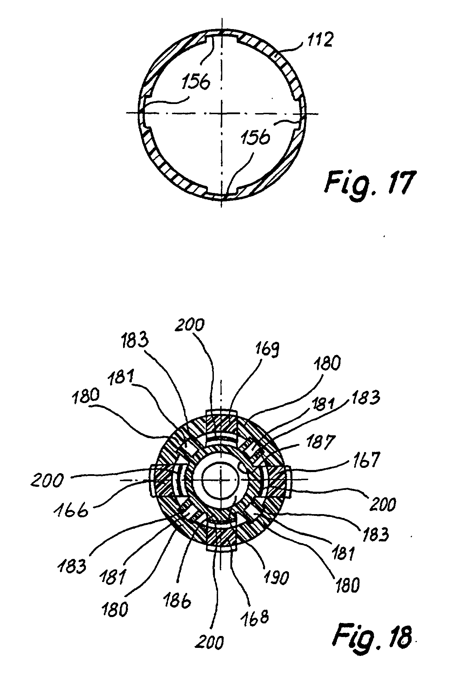

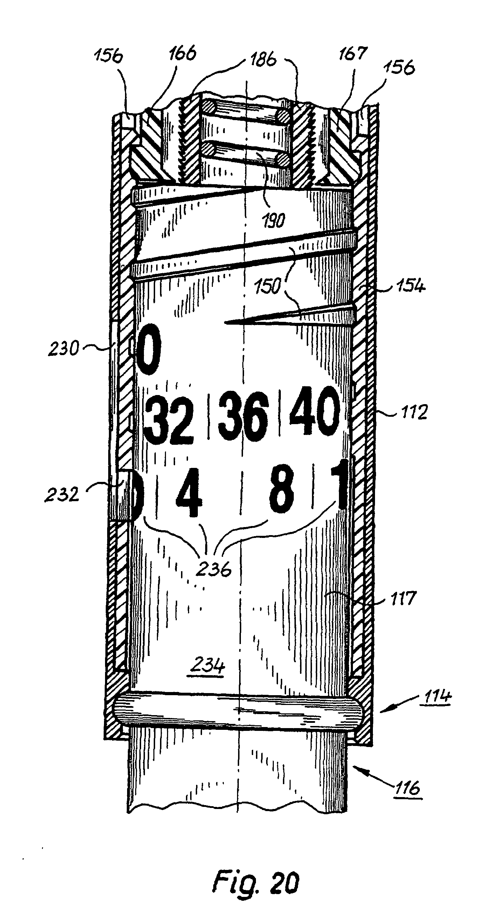

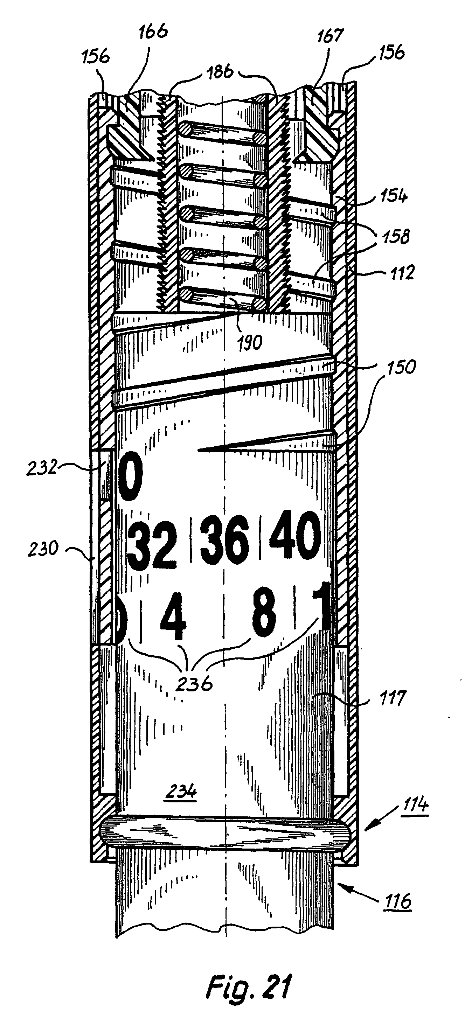

Zum Zwecke der Dosiseinstellung hat das proximale Gehäuseteil 116 einen distalen Abschnitt 117, der in das distale Gehäuseteil 112 hineinragt und auf seiner Außenseite mit einem Außengewinde 150 versehen ist, vgl. Fig. 15. Die Form dieses Gewindes 150 ergibt sich aus den Fig. 20 und 21. Es steht im Eingriff mit einem hierzu komplementären Innengewinde 152 (Fig. 14) einer als Dosierelement dienenden Gewindehülse 154, die in Längsnuten 156 (Fig. 17) des Gehäuseteils 112 axial verschiebbar geführt ist, also sich relativ zu letzterem nicht drehen kann.

Wird das proximale Gehäuseteil 116 relativ zum distalen Gehäuseteil 112 gedreht, so wird das Dosierelement 154 relativ zum Gehäuseteil 112 axial verschoben. Die Lage des Dosierelements 154 relativ zum Gehäuseteil 112 bestimmt deshalb die vorwählbare Injektionsdosis, die z.B. zwischen 2 und 60 Insulineinheiten verstellbar sein kann. Nachfolgend wird dies anhand der Fig. 20 und 21 näher erläutert.

Im Bereich des distalen Endes erweitert sich die zylindrische Innenseite 158 des Dosierelements 154 zu einer Nut 160, die in distaler Richtung durch einen Anschlag 162 (Fig. 14) in Form einer (Ring-)Schulter begrenzt ist und in proximaler Richtung durch eine profilierte Schulter 164, die im Querschnitt z.B. die Form eines Kreisabschnitts haben kann, oder generell: Einer schrägen Nockenfläche.

In der Praxis ist die Nut 160 nicht durchgehend, sondern hat periphere Unterbrechungen, um die Herstellung als Spritzgußteil zu erleichtem. Die Nut 160 und die Schulter 162 werden dort benötigt, wo eine Interaktion mit einem der nachfolgend beschriebenen Spannbacken 166 bis 169 stattfindet.

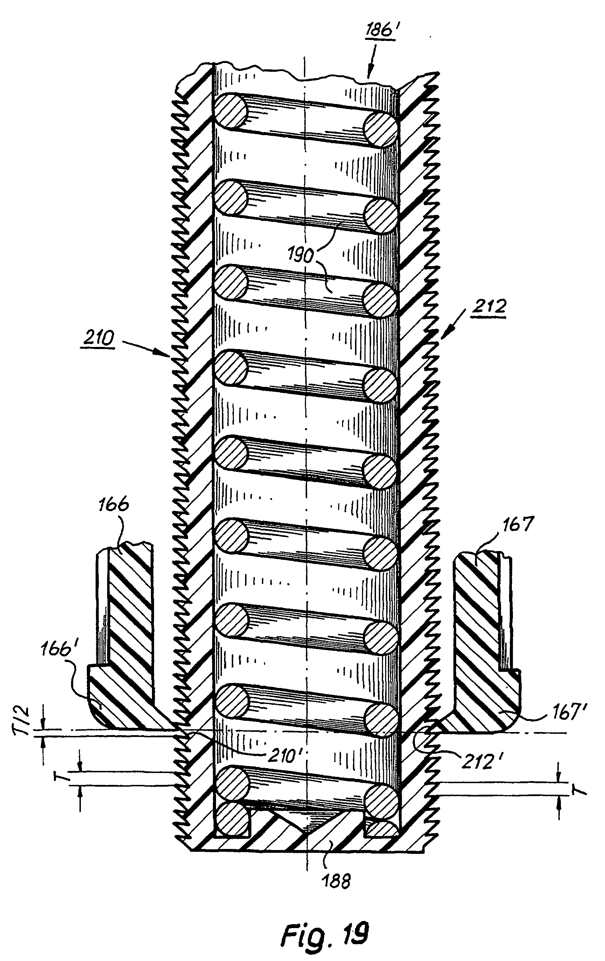

In der in Fig. 13 oder 14 dargestellten Stellung, die das Injektionsgerät 110 vor einer Injektion einnimmt, liegen in dieser Nut 160 die proximalen Enden von vier in Umfangsrichtung gleichmäßig verteilten Spannbacken 166, 167, 168, 169 eines Betätigungsglieds 170, dessen Form am besten aus den Fig. 16 und 18 hervorgeht. Diese Spannbacken sind einstückig ausgebildet mit einem Betätigungsknopf 172. Sie sind durch entsprechende Öffnungen 174, 176 eines ringförmigen Teils 178 durchgeführt, welches den distalen Abschluß des Gehäuseteils 112 bildet und mit diesem, z.B. durch eine Schnappverbindung, verbunden ist, vgl. Fig. 2. In den verschiedenen Längsschnitten sind nur die Spannbacken 166, 167 dargestellt. Ihre proximalen Enden sind dort mit 166', 167' bezeichnet. - Die Spannbacken 166 bis 169 sind, z.B. in den Längsnuten 156, im Gehäuses 112 in axialer Richtung geführt.

Es wird darauf hingewiesen, daß nicht notwendig eine Zahl von vier Spannbacken 166 bis 169 vorgesehen sein muß. Z.B. könnte man auch drei Spannbacken (nicht dargestellt) verwenden, die z.B. um 120° versetzt angeordnet sind. Naturgemäß würde man komplementär hierzu ein Auspreßglied 186 mit nur drei Zahnreihen 200 benötigen, von denen jede mit einer der drei Spannbacken zusammenwirkt. - Bevorzugt sollten sich die Kräfte, welche die Spannbacken auf das Auspreßglied 186 ausüben, gegenseitig im wesentlichen aufheben, d.h. wenn z.B. nur zwei Spannbacken verwendet werden, sollten sich diese etwa gegenüberliegen. Selbstverständlich ist im Rahmen der vorliegenden Erfindung auch die Verwendung nur einer Spannbacke nicht ausgeschlossen.

Das ringförmige Teil 178 hat gemäß Fig. 16 auf seiner Innenseite ein mit radialen Aussparungen (vgl. Fig. 18) versehenes Führungsrohr 180, dessen proximales Ende sich in Fig. 16 an der Stelle 182 befindet. Es hat auf seiner Innenseite vier Längsnuten 181, und diese dienen zur axialen Führung von radialen Vorsprüngen 183 eines im wesentlichen zylindrischen, hohlen Auspreßglieds 186, in dessen innerer zylindrischer Formhöhlung 187 sich, wie dargestellt, eine Druckfeder 190 befindet. Das Auspreßglied 186 kann auch als Kolbenstange bezeichnet werden.

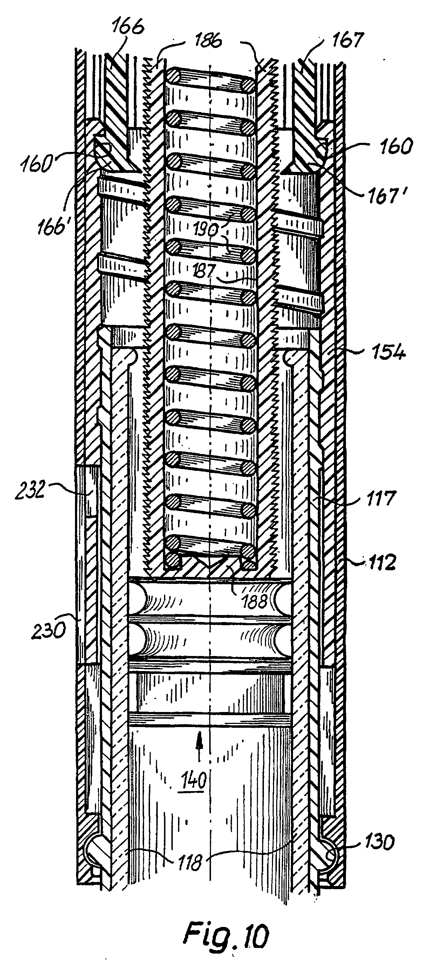

Die Druckfeder 190 ist mit ihrem proximalen Ende an einem proximalen Bodenteil 188 des Auspreßglieds 186 abgestützt, und mit ihrem distalen Ende am ringförmigen Teil 178. Diese Feder 190 hat eine schwache Vorspannung. Ihre Funktion ist nicht, wie man vielleicht glauben könnte, die Unterstützung des Injektionsvorgangs, sondern sie dient zur Nachführung des Auspreßglieds 186, damit dieses stets in der in Fig. 10 dargestellten Weise gegen den Kolben 140 anliegen kann, wenn die Spannbacken 166 bis 169 nicht in Eingriff mit dem Auspreßglied 186 stehen.

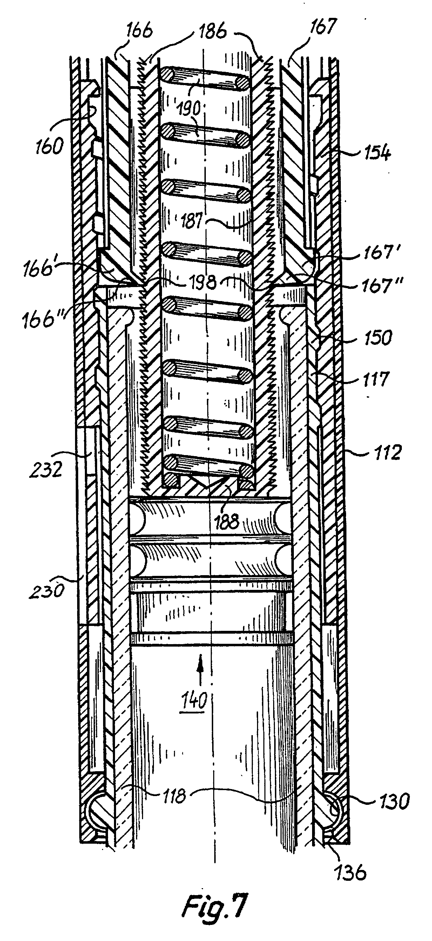

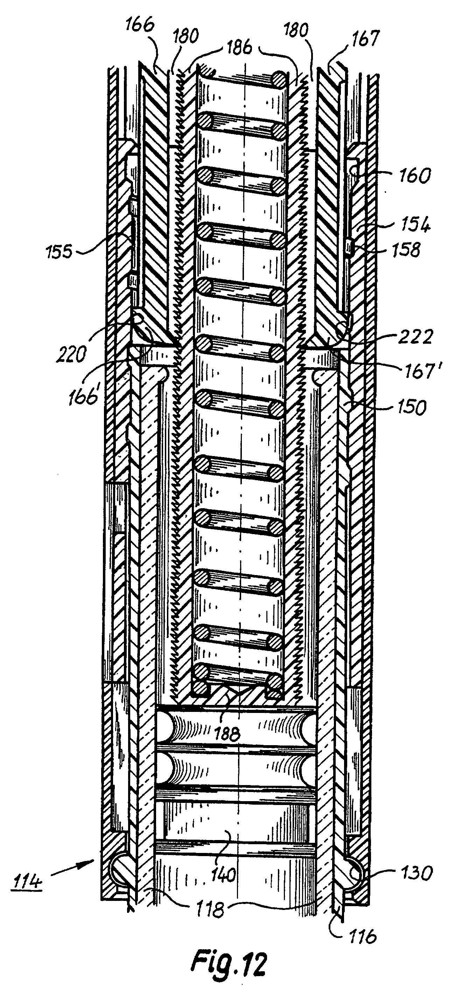

Wie ein Vergleich der Fig. 7 und 12 zeigt, bewegt sich bei jeder Injektion der Kolben 140 in proximaler Richtung weiter, und das Auspreßglied (Kolbenstange) 186 muß in jeder dieser Stellungen mit seinem Boden 188 gegen den Kolben 140 anliegen, ohne diesen jedoch zu verschieben, also mit einer Kraft, die unterhalb einer Losbrechkraft (z.B. 2...2,5 N) des Kolbens 140 liegt. Das bedeutet, daß die Feder 190 in ihrer maximal komprimierten Stellung, also bei gefüllter Karpule 118, keine Kraft erzeugen darf, die größer ist als diese Losbrechkraft, mit Vorteil aber kleiner, also bei diesem Beispiel maximal 1,5 N betragen wird. Mit anderen Worten kann man sagen, daß der Boden 188 des Auspreßglieds (Kolbenstange) 186 mit sanftem Druck gegen den Kolben 140 anliegt, aber ohne diesen dabei bewegen zu können. Die Feder 190 hat also nur eine Nachführfunktion und ist sehr schwach, bei niedriger Federkonstante.

Wie z.B. in Fig. 7 und 14 dargestellt, hat das Auspreßglied (Kolbenstange) 186 auf seiner Außenseite Vertiefungen 198 mit bevorzugt äquidistanten Abständen, hier in Form einer Verzahnung 200. Die proximalen Enden 166', 167' der Spannbacken 166, 167 haben Vorsprünge 166", 167" (Fig. 14), welche komplementär zu den Vertiefungen 198 ausgebildet sind. Dasselbe gilt völlig analog für die Spannbacken 168, 169 und die diesen zugeordneten, nicht dargestellten Verzahnungen des Auspressglieds 186.

Die Spannbacken 166 bis 169 haben eine Vorspannung radial nach außen, wie aus Fig. 16 hervorgeht, so daß sie in der Stellung, die der Injektor 110 direkt vor Beginn einer Injektion einnimmt, radial nach außen ausgelenkt sind und deshalb nicht in die Ausnehmungen 198 eingreifen. Dies zeigt Fig. 14, wo die freien Enden 166', 167' durch die beschriebene Vorspannung jeweils in die zugeordnete Nut 160 gepreßt werden und nicht in Eingriff mit den Zahnreihen 200 stehen. Man erhält so eine von der axialen Lage des Betätigungsglieds 170, 172 abhängige Antriebsverbindung zwischen den Spannbacken 166 bis 169 und dem Auspreßglied 186.

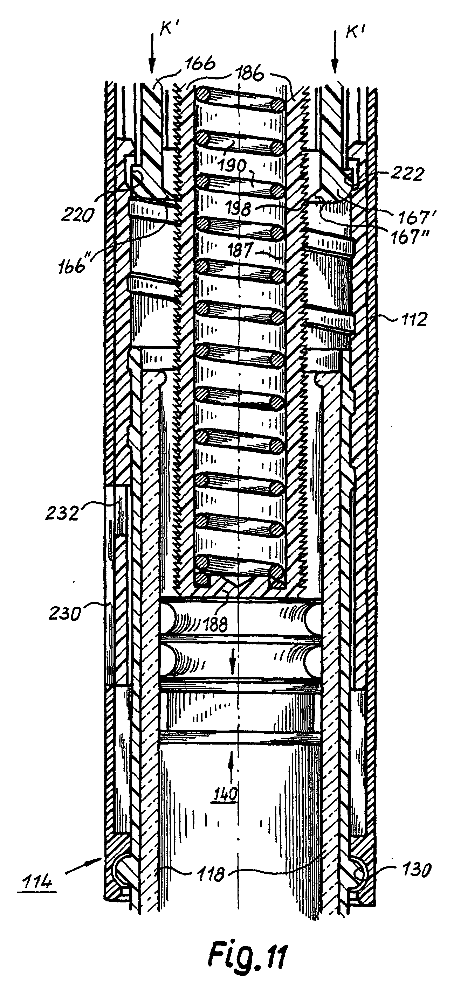

Wird nämlich in Fig. 13, 14 durch eine Kraft F der Betätigungsknopf 172 in proximaler Richtung verschoben, so werden die proximalen Enden 166', 167' der Spannbacken jeweils durch die Nockenfläche 164 der Nut 160 und die dazu komplementäre Form der Enden 166', 167' radial nach innen gepreßt, vgl. Fig. 11, und gelangen mit ihren Vorsprüngen 166", 167" in Eingriff mit der dem betreffenden Vorsprung gegenüberliegenden Ausnehmung 198, so daß zwischen dem Betätigungsglied 170, 172 und dem Auspreßglied (Kolbenstange) 186 eine Antriebsverbindung aktiviert wird, welche in der Stellung gemäß Fig. 13, 14 deaktiviert war.

Diese Antriebsverbindung bewirkt, daß die Bewegung des Betätigungsgliedes 170, 172 in proximaler Richtung (durch die Kraft F der Fig. 13) auf das Auspreßglied (Kolbenstange) 186 direkt, also formschlüssig, übertragen wird und es in proximaler Richtung verschiebt. Da es - durch die Kraft der schwachen Feder 190 - mit seinem Boden 188 bereits direkt gegen den Kolben 140 anliegt, wird diese Bewegung auch direkt auf den Kolben 140 übertragen und führt dazu, daß aus dem Behälter 118 über die Nadel 146 Flüssigkeit in der zuvor eingestellten Dosis ausgepreßt wird, sofern der Betätigungsknopf 172 durch die Kraft F so lange verschoben wird, bis er mit seinem proximalen Rand 172a (Fig. 13) gegen die distale Stirnseite 178a des ringförmigen Teils 178 anliegt, also bis zum Anschlag.

Hierbei ist darauf hinzuweisen, daß bei diesem Injektionsvorgang gemäß Fig. 12 jeweils die radiale Außenseite 220, 222 der proximalen Enden 166', 167' durch die Innenseite 155 des Dosierelements 154 nach Art einer Nockensteuerung radial nach innen und in Eingriff mit dem Auspreßglied (Kolbenstange) 186 gepreßt wird, so daß also - nach Verlassen der Nut 160 - die Antriebsverbindung zwischen Betätigungsglied 170 und Auspreßglied 186 ständig aufrechterhalten wird. Bevorzugt ist diese Antriebsverbindung eine formschlüssige Verbindung, doch wäre ggf. auch eine kraftschlüssige Verbindung möglich, wie dem Fachmann ohne weiteres ersichtlich ist. Alternativ könnte diese Verbindung z.B. auch durch Erregung eines Elektromagneten hergestellt werden.

### Arbeitsweise

Fig. 7 zeigt das Injektionsgerät 110 nach einer Injektion. Die Vorsprünge 166", 167" des Betätigungsglieds 170 stehen in Zwangseingriff mit einer zugeordneten Ausnehmung 198 der Zahnreihen 200.

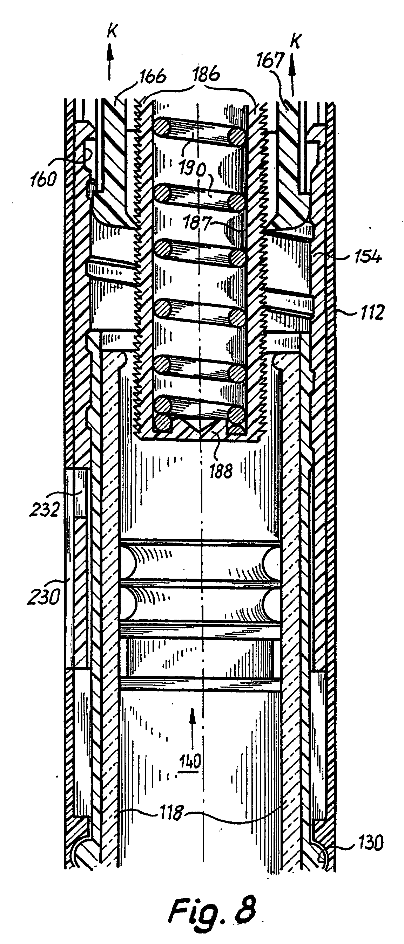

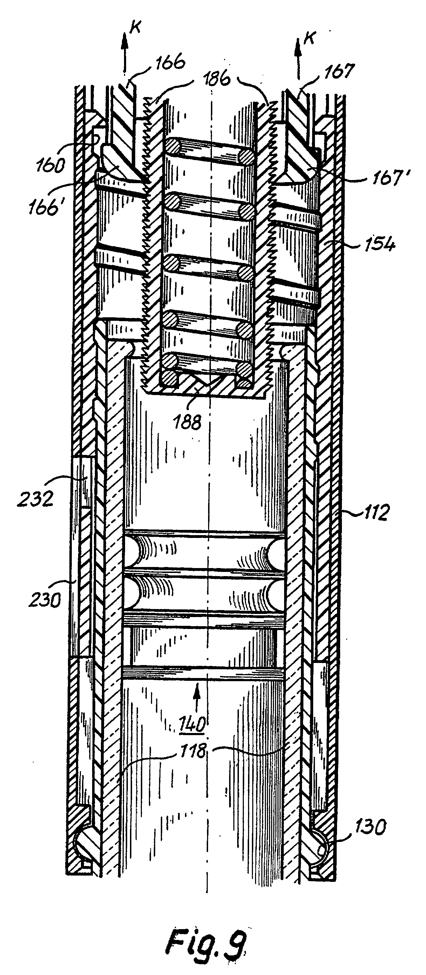

Gemäß Fig. 8 zieht nun der Benutzer am Betätigungsglied 170, indem er den Betätigungsknopf 172 mit einer Kraft K in distaler Richtung bewegt. Fig. 8 zeigt eine Zwischenstellung dieses Bewegungsvorgangs, und Fig. 9 eine weiter fortgeschrittene Zwischenstellung, bei der die Vorsprünge 166', 167' bereits fast die Nut 160 erreicht haben.

In Fig. 10 ist die Nut 160 erreicht. Die Spannbacken 166 bis 169 federn radial nach außen in diese Nut 160 und deaktivieren dadurch die Antriebsverbindung zum Auspreßglied (Kolbenstange) 186, so daß sich letzteres prompt unter der Wirkung der (schwachen) Feder 190 in proximaler Richtung bewegt, bis sein Boden 188 mit einer geringen Kraft gegen den Kolben 140 anliegt. Dies ist die bereits beschriebene Nachführbewegung des Auspreßglieds 186, und das Injektionsgerät 110 ist in dieser Stellung injektionsbereit.

Sollte der Benutzer den beschriebenen Vorgang bereits in der Stellung gemäß Fig. 8 oder Fig. 9 unterbrechen, so bewegt die Feder 190 das Auspreßglied 186 und das Betätigungsglied 170 in die Stellung gemäß Fig. 7 zurück, so daß in diesem Fall keine Injektion möglich ist. Eine Injektion wird vielmehr erst möglich, wenn die Stellung gemäß Fig. 10 erreicht ist, in der die Dosis aktiviert ist, die zuvor durch Verdrehen des Gehäuseteils 116 eingestellt worden war. Dies stellt ein wertvolles Sicherheitsmerkmal dar und verhindert, daß der Patient sich weniger als die von ihm vorgewählte Dosis injiziert.

Bei der Injektion sticht der Patient zuerst die Kanüle 146 (Fig. 13) in sein Unterhaut-Fettgewebe und drückt dann mit der Kraft F auf den Betätigungsknopf 172. Dabei bewegen sich (Fig. 11) die Enden 166', 167' der Spannbacken 166 bis 169 radial nach innen und kommen in Eingriff mit der ihnen jeweils gegenüberliegenden Ausnehmung 198. Dadurch wird die Kraft F auf das Auspreßglied 186 und über dieses auf den Kolben 140 übertragen, so daß die eingestellte Dosis aus dem Behälter 118 ausgepreßt und injiziert wird.

Fig. 12 zeigt den Abschluß dieses Vorgangs, d.h. das Ende einer Injektion mit der eingestellten Dosis. Wie bereits beschrieben, wird das Ende dann erreicht, wenn in Fig. 13 die Ringschulter 172a in Anlage gegen die Stirnseite 178a des Gehäuseteils 112 kommt.

Anschließend an die Injektion zieht der Patient die Nadel 146 aus dem Unterhaut-Fettgewebe und ersetzt sie durch eine neue, sterile Nadel, welche gewöhnlich gemäß Fig. 5 und 6 von einer sterilen Kappe 147 überdeckt ist.

Fig. 19 zeigt eine wesentliche Verbesserung, welche eine feinere Dosierung ermöglicht. Das Auspreßglied (Kolbenstange) 186' hat hier eine linke Zahnreihe 210 und eine rechte Zahnreihe 212. Beide haben eine identische Zahnteilung T, aber die Zahnreihen 210, 212 sind um eine halbe Zahnteilung gegeneinander in axialer Richtung versetzt, also um T/2, vgl. Fig. 19 . Da sich die Spannbacken 166, 167 ohne axiale Versetzung gegenüberliegen, greift also z.B. die Spannbacke 167 mit ihrem freien Ende 167' voll in eine Vertiefung 212' der Zahnreihe 212 ein, während das freie Ende 166' der Spannbacke 166 in der dargestellten Weise nur halb in die zugehörige Ausnehmung 210' der Zahnreihe 210 eingreift, d.h. im dargestellten Fall ist die rechte Spannbacke 167 wirksam, und stellt die Antriebsverbindung her. Umgekehrt kann es so sein, daß das freie Ende 166' der Spannbacke 166 voll in eine zugeordnete Ausnehmung 210' eingreift, während das freie Ende 167' nur halb in eine zugeordnete Ausnehmung 212' eingreift. - Es ist darauf hinzuweisen, daß Fig. 18 eine analoge Versetzung der Zahnreihen 200 im Schnitt zeigt, wie der Fachmann ohne weiteres erkennt.

Durch die Versetzung ist eine Dosiseinstellung mit der Hälfte einer Zahnteilung T möglich, also mit T/2, d.h. die Dosis kann bei dieser Variante in kleineren Schritten eingestellt werden, ohne daß hierzu kleinere Zähne 210, 212 erforderlich wären. Die Zahnreihen 210, 212 sind in Fig. 19 aus Gründen der Anschaulichkeit stark vergrößert dargestellt.

Wie man ohne weiteres erkennt, kann man beispielsweise auch drei verschiedene Zahnreihen verwenden und jede relativ zur anderen um T/3 versetzen, um eine noch feinere Einstellmöglichkeit zu erhalten. Ebenso ist es möglich, die freien Enden 166', 167' der Spannbacken 166, 167 relativ zueinander zu versetzen, z.B. um T/2, dafür aber nicht die Zahnreihen 210, 212. Solche und andere Varianten stehen dem Fachmann ohne weiteres zu Gebote.

Die Fig. 20 und 21 zeigen die Teile des Injektors, welche zur Dosiseinstellung vorgesehen sind. Das rohrartige Teil 116 ist in diesen beiden Figuren in der Draufsicht dargestellt, also nicht im Längsschnitt. Es ist im Gehäuse 112 über das Lager 114 drehbar gelagert, so daß es im Gehäuse 112 verdreht werden kann, ohne sich in diesem axial zu verschieben.

Auf seiner Außenseite hat der im Gehäuse 112 befindliche Teil 117 des rohrartigen Teils 116 ein Außengewinde 150 (Steilgewinde), dessen beide Gewindegänge bevorzugt trapezförmigen Querschnitt haben, und dieses Außengewinde 150 greift ein in das entsprechende Innengewinde 158 (Fig. 21) der als Dosierelement dienenden Gewindehülse 154, welche in den Längsnuten 156 des Gehäuses 112 axial geführt ist, sich also im Gehäuse 112 nicht drehen, sondem nur axial verschieben kann.

Das Gehäuse 112 hat ein längliches Fenster 230, dessen Form z.B. aus Fig. 1 hervorgeht und das sich in Längsrichtung des Gehäuses 112 erstreckt. Es dient zur Anzeige der eingestellten Injektionsdosis.

Ebenso hat das Dosierelement 154 ein Fenster 232, das axial kürzer ist als das Fenster 230, aber dieselbe Breite haben kann wie dieses.

Ferner sind auf der Außenseite 234 des rohrartigen Teils 116 in der dargestellten Weise Anzeigewerte 236 für die Injektionsdosis angebracht, also hier die Zahlen 0, 2, 4...60.

Das Fenster 232 ist so bemessen, daß es von diesen Anzeigewerten 236 immer nur einen einzigen anzeigen kann, z.B., wie in Fig. 1 dargestellt, den Anzeigewert "60".

Wie man aus Fig. 20 und 21 entnimmt, sind die Anzeigewerte 236 etwa schraubenförmig auf der Außenseite 234 des Teils 117 angebracht, d.h. mit zunehmender Dosis "wandert" die Anzeige im Fenster 230 in distaler Richtung, da sich die Gewindehülse 154 in distaler Richtung im Gehäuse 112 verschiebt.

Fig. 20 zeigt die Stellung des Dosierelements 154 für die Injektionsdosis "0"; diese Stellung ist auch in Fig. 5 dargestellt. Fig. 21 zeigt die Stellung des Dosierelements 154 für die maximale Injektionsdosis, also z.B. "60"; diese ist auch in Fig. 1 dargestellt. Ein Vergleich der Fig. 20 und 21 zeigt die unterschiedliche Lage des Dosierelements 154 relativ zum Gehäuse 112, ebenso die unterschiedliche Lage des Fensters 232 relativ zum Fenster 230.

Hier sei nochmals darauf hingewiesen, daß eine einmalige Dosisvorwahl im Fenster 232, also z.B. vier Insulineinheiten ("4"), für alle nachfolgenden Injektionen in gleicher Weise gilt, d.h. wenn diese Dosis unverändert beibehalten wird, genügt eine einmalige Einstellung, was für den Patienten eine erhebliche Erleichterung darstellt, da er sich - bei konstanter Dosis - vor einer Injektion nicht mehr um die Dosiseinstellung zu kümmern braucht.

Naturgemäß sind im Rahmen der vorliegenden Erfindung vielfache Abwandlungen und Modifikationen möglich, z.B. die Ausgestaltung eines erfindungsgemäßen Injektors als sogenannter Vollautomat mit vollautomatischem Ablauf der Injektion.

## Patentansprüche

1. Injektionsgerät zum Injizieren einer einstellbaren Dosis einer Injektionsflüssigkeit,
mit einem distalen Gehäuseteil (112) und einem proximalen Gehäuseteil (116), welche relativ zueinander drehbar gelagert sind, aber axial relativ zueinander nicht verschiebbar sind,
wobei das proximale Gehäuseteil (116) einen proximalen Abschnitt aufweist, der aus dem distalen Gehäuseteil (112) herausragt, und einen distalen Abschnitt (117), welcher sich in das distale Gehäuseteil (112) erstreckt und ein Außengewinde (150) aufweist,
mit einem Dosierglied (154), welches im distalen Gehäuseteil (112) axial verschiebbar und nicht verdrehbar angeordnet ist, durch seine Lage relativ zum distalen Gehäuseteil (112) die Injektionsdosis beeinflusst, und mit einem Innengewinde (152) versehen ist, welches mit dem Außengewinde (150) des distalen Abschnitts (117) des proximalen Gehäuseteils (116) in Eingriff steht,
um durch Verdrehen des proximalen Gehäuseteils (116) relativ zum distalen Gehäuseteil (112) eine axiale Verschiebung des Dosierglieds (154) im distalen Gehäuseteil (112) und dadurch eine Einstellung der Injektionsdosis zu ermöglichen.

2. Injektionsgerät nach Anspruch 1, bei welchem auf der Außenseite des distalen Abschnitts (117) des proximalen Gehäuseteils (116) Anzeigewerte (236) für die Injektionsdosis vorgesehen sind.

3. Injektionsgerät nach Anspruch 2, mit einem ersten Fenster (230), welches im distalen Gehäuseteil (112) vorgesehen ist,
und mit einem zweiten Fenster (232), welches unterhalb des ersten Fensters (230) im Dosierglied (154) vorgesehen ist,
um durch das erste Fenster (230) und durch das zweite Fenster (232) einen Anzeigewert (236) für die eingestellte Injektionsdosis auf der Außenseite (234) des distalen Abschnitts (117) des proximalen Gehäuseteils (116) sichtbar zu machen.

4. Injektionsgerät nach Anspruch 2, bei welchem das im Dosierglied (154) vorgesehene Fenster (232) so bemessen ist, dass es im wesentlichen der von einem Anzeigewert (236) für die Injektionsdosis eingenommenen Fläche entspricht und mit seiner Peripherie benachbarte Anzeigewerte überdeckt.

5. Injektionsgerät nach Anspruch 2, bei welchem das im distalen Gehäuseteil (112) vorgesehene Fenster (230) die Form einer länglichen Ausnehmung aufweist, deren Längsachse sich mindestens nahezu in Längsrichtung des Injektionsgeräts (110) erstreckt.

6. Injektionsgerät nach Anspruch 1, bei welchem der proximale Abschnitt des proximalen Gehäuseteils (116), welcher aus dem distalen Gehäuseteil (112) herausragt, als Halter für eine Kartusche (118) mit der zu injizierenden Flüssigkeit ausgebildet ist.

## Claims

1. Injection device for injecting an adjustable dose of an injection liquid,
comprising a distal housing part (112) and a proximal housing part (116) which are rotatably journalled relative to one another but are not displaceable axially relative to one another,
the proximal housing part (116) comprising a proximal portion which projects out of the distal housing part (112), and a distal portion (117) which extends into the distal housing part (112) and comprises an external thread (150),
further comprising a dosing member (154) which is arranged so as to be displaceable axially but not rotatable in the distal housing part (112), and affects the injection dose through its position relative to the distal housing part (112), and is provided with an internal thread (152) which is engaged with the external thread (150) of the distal portion (117) of the proximal housing part (116),
in order to allow axial displacement of the dosing member (154) in the distal housing part (112) and thus adjustment of the injection dose by rotation of the proximal housing part (116) relative to the distal housing part (112).

2. Injection device according to claim 1, in which indicating values (236) for the injection dose are provided on the outside of the distal portion (117) of the proximal housing part (116).

3. Injection device according to claim 2, comprising a first window (230) which is provided in the distal housing part (112),
and a second window (232) which is provided underneath the first window (230) in the dosing member (154),
in order to make an indicating value (236) for the set injection dose on the outside (234) of the distal portion (117) of the proximal housing part (116) visible through the first window (230) and through the second window (232).

4. Injection device according to claim 2, in which the window (232) provided in the dosing member (154) is dimensioned so that it essentially corresponds to the area taken up by one indicating value (236) for the injection dose and covers neighbouring indicating values with its periphery.

5. Injection device according to claim 2, in which the window (230) provided in the distal housing part (112) has the shape of an elongated recess the longitudinal axis of which extends at least almost in the longitudinal direction of the injection device (110).

6. Injection device according to claim 1, in which the proximal portion of the proximal housing part (116) which portion projects out of the distal housing part (112) and is adapted as a holder for a cartridge (118) containing the liquid to be injected.

## Revendications

1. Dispositif d'injection pour injecter une dose variable d'un liquide à injecter, comprenant une partie de boîtier distale (112) et une partie de boîtier proximale (116) qui sont assemblées de manière à pouvoir tourner l'une par rapport à l'autre, mais à ne pas pouvoir coulisser axialement l'une par rapport à l'autre, sachant que la partie de boîtier proximale (116) présente un segment proximal faisant saillie de la partie de boîtier distale (112) et un segment distal (117) qui pénètre dans la partie de boîtier distale (112) et présente un filetage extérieur (150), comprenant également un organe de dosage (154) qui est placé dans la partie de boîtier distale (112) de façon à pouvoir coulisser axialement et fixe en rotation, qui influe sur la dose à injecter par sa position par rapport à la partie de boîtier distale (112) et qui est muni d'un taraudage (152) en prise avec le filetage (150) du segment distal (117) de la partie de boîtier proximale (116), afin de permettre un déplacement axial de l'organe de dosage (154) dans la partie de boîtier distale (112) en faisant tourner la partie de boîtier proximale (116) par rapport à la partie de boîtier distale (112), et par là même un réglage de la dose à injecter.

2. Dispositif d'injection selon la revendication 1, dans lequel des valeurs d'affichage (236) correspondant à la dose à injecter sont prévues sur l'extérieur du segment distal (117) de la partie de boîtier proximale (116).

3. Dispositif d'injection selon la revendication 2, comprenant une première fenêtre (230) prévue dans la partie de boîtier distale (112) et une seconde fenêtre (232) prévue dans l'organe de dosage (154), sous la première fenêtre (230), pour faire apparaître dans la première (230) et la seconde (232) fenêtre, sur l'extérieur (234) du segment distal (117) de la partie de boîtier proximale (116), une valeur d'affichage (236) correspondant à la dose à injecter.

4. Dispositif d'injection selon la revendication 2, dans lequel la fenêtre (232) prévue dans l'organe de dosage (154) est dimensionnée de telle sorte qu'elle correspond pour l'essentiel à la surface occupée par une valeur d'affichage (236) d'une dose à injecter et qu'elle recouvre avec son pourtour des valeurs d'affichage voisines.

5. Dispositif d'injection selon la revendication 2, dans lequel la fenêtre (230) prévue dans la partie de boîtier distale (112) a la forme d'un évidement oblong dont l'axe longitudinal s'étend au moins approximativement dans la direction longitudinale du dispositif d'injection (110).

6. Dispositif d'injection selon la revendication 1, dans lequel le segment proximal de la partie de boîtier proximale (116), qui fait saillie de la partie de boîtier distale (112), est réalisé sous forme de support pour une cartouche (118) contenant le liquide à injecter.
